# FASCICULE DE BREVET EUROPEEN

(11) **EP 2 531 483 B1**
(45) Date de publication et mention de la délivrance du brevet: **07.10.2015**
(21) Numéro de dépôt: 11713034.4
(22) Date de dépôt: 03.02.2011
(51) Int. Cl.: C07C 67/08, C07C 69/14

(54) **PROCEDE DE PREPARATION D'UN ESTER D'ACIDE CARBOXYLIQUE**
VERFAHREN ZUR HERSTELLUNG EINES CARBOXYLSÄUREESTERS
METHOD FOR PREPARING A CARBOXYLIC ACID ESTER

(30) Priorité: 04.02.2010 FR 1050768
(43) Date de publication de la demande: 12.12.2012
(73) Titulaire: Rhodia Poliamida E Especialidades Ltda, Sao Paulo - SP (BR)
(72) Inventeur: MARENCO, Carlos, Eduardo, 13105-800 - Campinas (SP) (BR); MARTINS, Wilson, 13085-260 - Campinas (SP) (BR); TRESMONDI, Alexandre, 13023-200 - Campinas (SP) (BR); SCHWARTZ, Joël, F-69300 Caluire (FR)
(74) Mandataire: Ridray, Annabelle
(86) Numéro de dépôt international: PCT/IB2011/000172
(87) Numéro de publication internationale: WO 2011/095872

(56) Documents cités:
- EP-A1- 1 013 632
- WO-A1-98/25876
- DE-A1- 3 636 754

## Description

La présente invention concerne un procédé de fabrication d'un ester d'acide carboxylique.

L'invention vise notamment la préparation des esters de l'acide acétique et plus particulièrement l'acétate d'éthyle.

Les esters de l'acide acétique, en particulier l'acétate d'éthyle, sont utilisés généralement comme solvants organiques. En particulier, l'acétate d'éthyle est utilisé notamment dans le domaine de la cosmétique, de la parfumerie, dans les colles, peintures et vernis.

Selon l'application visée, une pureté plus ou moins élevée est demandée et il est courant de requérir que la quantité d'acide acétique présent dans l'acétate d'éthyle soit inférieure à 0,01 % en masse.

Ainsi, les procédés de fabrication de l'acétate d'éthyle doivent conduire à un produit de qualité. Etant donné que l'acétate d'éthyle est un produit de consommation courante, à gros volume, il importe que son procédé de fabrication soit le plus performant possible, en termes de productivité et de bilan énergétique.

Il est décrit selon US 4481146, un procédé de préparation d'acétate d'éthyle dans lequel le rapport molaire entre l'acide acétique et l'alcool est compris entre 1 et 5. DE3636754 décrit la préparation d'esters d'acide carboxylique par réaction entre un acide carboxylique et un alcool en C3-C5, avec un excès d'acide allant de 5 à 12 moles d'acide par moles d'alcool.

Toutefois, la productivité de ces procédés ainsi que la réduction des coûts énergétiques de production peuvent être améliorés.

L'objectif de la présente invention est de fournir un procédé perfectionné de préparation d'un ester d'acide carboxylique, en termes d'économie de procédé.

La présente invention a donc pour objet un procédé de fabrication d'un ester d'acide carboxylique par réaction entre un acide carboxylique et un alcool, notamment en présence d'un catalyseur acide, dans lequel la réaction est conduite de telle sorte que le ratio molaire acide carboxylique/alcool est au moins égal à 16.

Conformément au procédé de l'invention, on effectue une réaction d'estérification de l'acide acétique, par un alcool de préférence en présence d'un catalyseur acide. Il a été trouvé selon l'invention, que dès lors que le ratio molaire acide carboxylique/alcool est très élévé, c'est-à-dire au moins égal à 16, le procédé est amélioré en ce qui concerne les coûts énergétiques de fonctionnement.

En outre, les exigences de teneur maximale en acide carboxylique dans l'ester d'acide carboxylique obtenu sont respectées.

Intervient dans le procédé de l'invention, un acide carboxylique, un alcool et de préférence un catalyseur acide.

Le procédé de l'invention convient tout à fait bien à l'acide acétique.

L'acide carboxylique est avantageusement introduit pur ou en solution aqueuse très concentrée. Le procédé de l'invention n'exclut pas la présence d'eau dans l'acide carboxylique. Cependant, il est préférable d'utiliser de l'acide carboxylique pur du fait de la nécessité ultérieure d'éliminer l'eau présente dans l'ester d'acide carboxylique obtenu en fin de procédé.

L'alcool est choisi parmi l'éthanol, le butanol, le n-propanol et le cyclohexanol, de péférence l'éthanol.

Selon l'invention, le ratio molaire entre l'acide carboxylique et l'alcool est au moins égal à 16.

La borne supérieure, pour des raisons économiques, est avantageusement choisie inférieure à 25, de préférence inférieure à 20. Ainsi, le ratio molaire entre l'acide carboxylique et l'alcool est de préférence compris entre 16 et 25, et plus préférentiellement entre 16 et 20. Le ratio précisément défini correspond au ratio molaire des réactifs en début de réaction.

Le catalyseur intervenant dans le procédé de l'invention est de préférence un acide protonique.

Selon un premier mode, le catalyseur est un catalyseur acide hétérogène. Les catalyseurs acides hétérogènes de l'invention sont préférentiellement des résines sulfoniques ou des zéolithes. Les zéolithes qui peuvent être utilisés sont par exemple celles citées dans la demande WO2007/099071. Les résines qui conviennent à la présente invention peuvent être constituées d'un squelette polystyrénique ou polyacrylique qui porte des groupes fonctionnels sulfoniques. Ainsi, on peut mettre en oeuvre les résines sulfoniques existant sur le marché, résines commercialisées sous différentes dénominations commerciales. On peut citer, entre autres, les résines d'estérification suivantes : Amberlyst® 15 de Rhom Haas, Amberlite® IR-120 H de Rhom Haas, Lewatit® 2631 de Bayer et K1431 de Bayer. L'acidité de ces résines est par exemple comprise entre 1 et 10 eq/kg (H+). Ces résines sont notamment mises en oeuvre en lit fixe ou fluidisé, de préférence en lit fixe.

Selon un second mode, le catalyseur est un catalyseur acide fort homogène. Ce second mode est un mode préféré de réalisation de l'invention.

Par acide fort, on désigne dans la présente invention, un acide présentant un pKa dans l'eau inférieur à 2, de préférence inférieur à 1.

Le pKa est défini comme suit : pKa = - log Ka, Ka étant la constante de dissociation ionique du couple acide/base à température ambiante (généralement 25°C), lorsque l'eau est utilisée comme solvant.

Parmi les acides répondant à cette définition, il est préférable d'utiliser un acide ne conduisant pas à des réactions parasites gênantes pour le procédé d'estérification et en particulier ne présentant pas de caractère oxydant, comme l'acide nitrique.

On peut citer comme acide fort homogène plus particulièrement l'acide sulfurique, les acides sulfoniques et leurs mélanges.

Comme acides sulfoniques, on peut mentionner notamment l'acide fluorosulfonique, l'acide chlorosulfonique ou l'acide trifluorométhanesulfonique, l'acide méthanesulfonique, l'acide éthanesulfonique, l'acide camphène-sulfonique, l'acide benzènesulfonique, les acides toluènesulfoniques, les acides xylènesulfoniques, les acides naphtalènesulfoniques.

Parmi ces acides, le catalyseur préféré est choisi parmi l'acide para-toluènesulfonique ou l'acide méthanesulfonique, de préférence l'acide méthanesulfonique.

La quantité de catalyseur introduite est telle que la quantité présente dans le réacteur est de préférence comprise entre 0,1 et 2 % en masse par rapport au milieu réactionnel.

Il est possible d'introduire d'autres composés dans la réaction, par exemple des inhibiteurs de corrosion. Il peut s'agir notamment de sulfate de cuivre(II).

Conformément au procédé de l'invention, la réaction d'estérification peut être conduite selon un mode continu ou discontinu.

Selon un mode préféré de réalisation de l'invention, le procédé est un procédé en continu.

Selon l'invention, on met en oeuvre les étapes suivantes :
a. on fait réagir l'acide carboxylique et l'alcool, en présence d'un catalyseur acide, à une température au moins égale à 50°C, et à une pression telle que le mélange réactionnel est à l'état liquide,
b. le mélange réactionnel liquide précédemment obtenu est ensuite soumis à une opération de distillation afin d'obtenir :
   ∘ un flux vapeur comprenant majoritairement l'ester de l'acide carboxylique, en tête de distillation et,
   ∘ un milieu comprenant majoritairement de l'acide carboxylique en pied de distillation,
c. on soutire le milieu en pied de distillation et on le réintroduit en début de réaction a.

Dans le procédé de l'invention, l'acide carboxylique et l'alcool peuvent être introduits seuls ou en mélange. De préférence, l'acide carboxylique et l'alcool sont introduits en mélange.

De façon avantageuse, la température de la réaction est comprise entre 50 et 150°C, de préférence entre 100 et 130°C.

La réaction est préférentiellement conduite à pression atmosphérique. Une pression légèrement supérieure ou inférieure à la pression atmosphérique peut également convenir. La réaction est avantageusement conduite à une pression telle que le mélange réactionnel est à l'état liquide. Ainsi, le procédé de l'invention peut être mis en oeuvre, par exemple, à une pression absolue comprise entre 0,5 et 5 bars absolus, et encore plus préférentiellement entre 1,5 et 5 bars.

De façon avantageuse, le mélange réactionnel obtenu en fin de réaction est soumis à une opération de distillation. Cette opération de distillation est de préférence conduite dans une colonne de distillation. Le point d'alimentation où est introduit le mélange réactionnel est en général sensiblement à mi-hauteur de la colonne de distillation. Il peut être également situé plus bas et à une hauteur comprise entre la mi-hauteur et le pied de la colonne.

La température en pied de distillation est de préférence comprise entre 50 et 150°C, de préférence entre 100 et 130°C.

La pression définie en tête de distillation est de préférence comprise entre 0,5 et 5 bars absolus, avantageusement la pression en tête de distillation est comprise entre 1 et 2 bars absolus.

Cette opération de distillation permet l'obtention d'un flux vapeur comprenant majoritairement l'ester de l'acide carboxylique en tête de distillation et un milieu en pied de distillation comprenant majoritairement de l'acide carboxylique.

Par « majoritairement », on entend que le milieu est constitué au moins à 75 % en masse, de préférence au moins à 85 % en masse du composé concerné.

Dans le procédé de l'invention, le flux vapeur en tête de distillation comprend notamment :
- 75 à 98 % en masse d'ester de l'acide carboxylique,
- 0 à 17 % en masse d'eau,
- 0 à 8 % en masse d'alcool,
- des traces d'acide carboxylique,

Le flux vapeur en tête de distillation comprend préférentiellement :
- 85 à 95 % en masse d'ester de l'acide carboxylique,
- 0 à 10 % en masse d'eau,
- 0 à 5 % en masse d'alcool,
- des traces d'acide carboxylique.

Par « traces d'acide carboxylique », on entend moins de 0,02% en masse de préférence entre 0,001 et 0,02% en masse d'acide carboxylique.

Dans le procédé de l'invention, le milieu en pied de distillation comprend notamment :
- 75 à 98 % en masse d'acide carboxylique,
- 0 à 15 % en masse d'ester de l'acide carboxylique,
- 0 à 10 % en masse d'eau,
- 0 à 3 % en masse d'alcool.

Le milieu en pied de distillation comprend préférentiellement :
- 80 à 95 % en masse d'acide carboxylique,
- 2 à 15 % en masse d'ester de l'acide carboxylique,
- 2 à 8 % en masse d'eau,
- 0,1 à 2 % en masse d'alcool.

Le milieu en pied de distillation peut en outre contenir jusqu'à 2% en masse de catalyseur.

Selon ce mode particulier de réalisation de l'invention, le milieu en pied de distillation est soutiré et recyclé par réintroduction en amont ou au cours de la réaction, de préférence en amont.

Par « en amont » on entend que le milieu en pied de distillation est réintroduit dans le mélange d'acide carboxylique et d'alcool avant qu'il ait réagi.

Le ratio entre le débit du flux de recyclage et le débit d'alimentation du mélange réactionnel (acide carboxylique + alcool) est avantageusement compris entre 4 et 20, de préférence entre 5 et 15.

Dans ce mode de réalisation, le ratio molaire acide carboxylique/alcool est soit le ratio dans le mélange comprenant l'acide carboxylique et l'alcool avant la réaction lorsque les réactifs sont introduits en mélange, soit le ratio en début de la réaction lorsque les réactifs sont introduits séparément dans la réaction. Ce ratio tient compte de l'apport en acide carboxylique et en alcool provenant du recyclage.

De façon avantageuse, le ratio molaire acide carboxylique/alcool à la sortie de la réaction est compris entre 40 et 120, de préférence entre 60 et 100.

Le mélange réactionnel, préférentiellement liquide, issu de la réaction d'estérification est distillé et permet d'obtenir en tête de colonne un flux vapeur. On refroidit le flux vapeur et le transforme sous forme liquide en abaissant sa température à une température par exemple comprise entre 15°C et 40°C par passage dans un ou plusieurs condenseurs.

Le flux liquide ainsi obtenu peut ensuite être envoyé vers un moyen de séparation de phases liquides, de préférence un décanteur séparant la phase organique contenant majoritairement l'ester de l'acide carboxylique et la phase aqueuse contenant majoritairement de l'eau.

La phase organique est, de façon avantageuse, réintroduite en partie en tête de distillation afin d'assurer le fonctionnement au reflux de la colonne. Le taux de reflux est de préférence compris entre 1 et 8 avantageusement entre 2 et 6. Le restant de la phase organique constitue l'ester de l'acide carboxylique attendu et peut être retraité, notamment par distillation, pour éliminer les traces résiduelles d'eau et d'alcool.

La phase aqueuse issue de la séparation est traitée, par exemple par distillation, de telle sorte que l'on récupère une phase comprenant majoritairement de l'alcool et une phase comprenant majoritairement de l'eau. La phase comprenant majoritairement de l'alcool peut avantageusement être recyclée par réintroduction en début de réaction.

Selon un autre aspect plus spécifique, la présente invention a également pour objet un dispositif pour la mise en oeuvre du procédé de l'invention.

Ce dispositif, qui se présente le plus souvent sous la forme d'une installation de dimensions industrielles, comprenant :
- un réacteur,
- une colonne de distillation dont :
   ∘ la partie haute est reliée à des moyens pour traiter le flux vapeur issu de la tête de colonne de distillation, à savoir au moins un condenseur dont la sortie est reliée à un séparateur de phase liquide/liquide, de préférence un décanteur et,
   ∘ la partie basse est reliée à des moyens pour soutirer et recycler le flux liquide issu du pied de la colonne de distillation vers le réacteur.

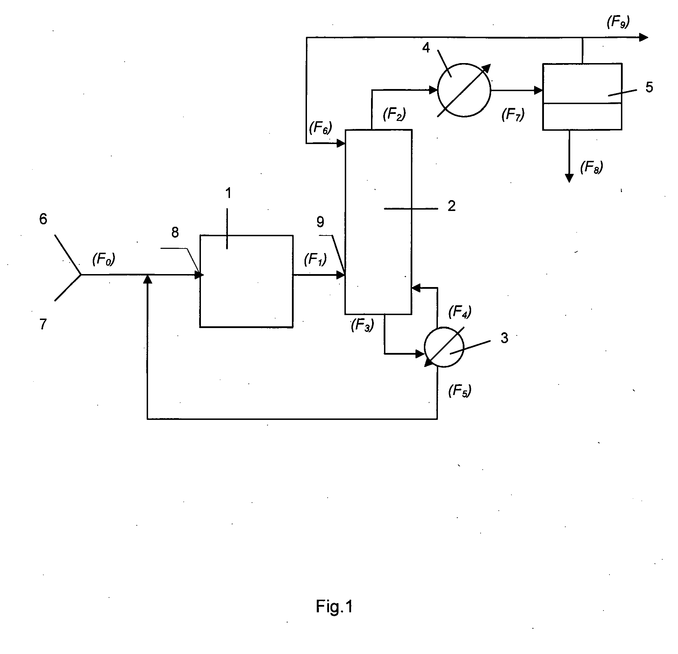

L'invention sera encore explicitée davantage par le biais de la description qui suit, faite en référence à la figure 1.

La figure 1 est une représentation schématique d'un dispositif préféré pour la mise en oeuvre du procédé selon l'invention comprenant un réacteur 1, une colonne de distillation 2 dont la partie haute est reliée à des moyens pour traiter le flux vapeur issu de la colonne de distillation 2, à savoir au moins un condenseur 4 dont la sortie est reliée à un décanteur 5.

L'alcool 6 et l'acide carboxylique 7 forment un flux préférentiellement liquide (*F₀*) qui est introduit en 8 dans un réacteur 1.

Le réacteur est de préférence adiabatique. Il peut être du type parfaitement agité ou du type piston, de préférence du type piston.

Le flux préférentiellement liquide *(F₁)* issu de la réaction est introduit en 9 dans une colonne de distillation 2.

Cette étape vise à obtenir en pied un flux liquide *(F₃)* comprenant majoritairement l'acide carboxylique et en tête un flux vapeur *(F₂)* comprenant majoritairement l'ester de l'acide carboxylique attendu.

L'homme du métier est parfaitement en mesure de choisir les moyens à mettre en oeuvre en fonction de la séparation à effectuer.

On rappellera simplement ce qui suit. La taille (notamment le diamètre) des colonnes de distillation dépend du flux circulant et de la pression interne. Leur dimensionnement se fera donc principalement suivant le débit de mélange à traiter. Le paramètre interne qu'est le nombre d'étages théoriques est déterminé notamment par la pureté du composé de départ et les puretés des produits devant être obtenues en tête et en pied de distillation.

On précisera que la colonne peut être garnie indifféremment de plateaux ou de garnissage ordonné ou tissé, comme cela est parfaitement connu de l'homme du métier.

L'installation étant déterminée, l'homme du métier ajuste les paramètres de fonctionnement de la colonne.

Ainsi, la colonne de distillation pourra être avantageusement, mais non limitativement, une colonne ayant les spécifications suivantes :
- nombre d'étages théoriques : de 1 à 40, de préférence de 2 à 20,
- taux de reflux R compris entre 1 et 8, de préférence entre 2 et 6.
Le taux de reflux est défini par le rapport du débit de matière réinjectée de la tête de la colonne vers l'intérieur de la colonne et du débit de phase organique sortant effectivement du décanteur.

Pour effectuer la distillation, l'apport des calories en pied de colonne peut être fait notamment par un échangeur tubes calandre, un échangeur à plaques, un échangeur à serpentin ou par tout autre dispositif équivalent. Le chauffage peut se faire à la vapeur ou par un fluide caloporteur.

Un mode de réalisation préféré consiste à chauffer le mélange en pied de distillation dans un échangeur thermique 3 par prélèvement d'un flux *(F₄)* en pied qui circule en boucle. Plus précisément, le flux *(F₃)* sort en pied de distillation et une fraction *(F₄)* traverse de bas en haut un échangeur thermique et en sortie d'échangeur est introduit sous forme d'un mélange liquide vapeur dans la partie inférieure de la colonne de distillation.

Un autre mode de réalisation consiste à effectuer une circulation forcée du flux *(F₃)* dans l'échangeur à l'aide d'une pompe.

Le flux *(F₅)* en sortie d'échangeur est réacheminé en amont par exemple par l'intermédiaire de pompes. Le ratio du débit du flux *(F₅)* sur le débit du flux *(F₀)* est de préférence compris entre 4 et 20.

Le flux vapeur *(F₂),* en tête de colonne, comprenant majoritairement l'ester de l'acide carboxylique est condensé de manière à récupérer un flux liquide dont une fraction *(F₆)* est introduite, latéralement en tête de colonne, pour assurer le reflux dans la colonne et l'autre fraction (*F₉*) peut être traitée dans une étape ultérieure de purification de l'ester de l'acide carboxylique.

En tête de distillation, la récupération du flux comprenant essentiellement l'ester de l'acide carboxylique à partir du flux *(F₂)* se fait par condensation, par exemple par passage dans un ou plusieurs condenseurs 4.

On refroidit la phase vapeur (*F₂*) et la transforme sous forme liquide par refroidissement en abaissant sa température à une température comprise par exemple entre 15°C et 40°C.

Cette opération est conduite par passage dans un condenseur qui est un appareil classique par exemple un échangeur tubulaire alimenté par un fluide caloporteur (généralement de l'eau) maintenu à une température voisine de la température de refroidissement choisie.

Le nombre et la taille des condenseurs sont choisis en fonction des capacités réfrigérantes des liquides de refroidissement circulant dans les condenseurs.

Dans le cas de condenseurs en série, la phase vapeur en sortie du premier condenseur est introduite dans le deuxième condenseur.

On récupère en sortie du ou des condenseurs, le flux liquide *(F₇)*.

Le flux liquide *(F₇)* est introduit dans un décanteur 5 qui sépare la phase aqueuse *(F₈)* de la phase organique *(F₉)*.

La phase *(F₈)* peut être retraitée par passage dans un dispositif d'entrainement (non représenté) permettant de récupérer d'une part une phase comprenant majoritairement de l'eau et d'autre part une phase comprenant majoritairement de l'alcool et de l'ester de l'acide carboxylique en mélange.

L'ester attendu est présent dans le flux *(F₉)*, et peut éventuellement être purifié notamment par distillation pour éliminer les quantités résiduelles d'alcool et d'eau.

Le procédé de l'invention est particulièrement intéressant en raison des avantages qu'il procure.

Un des avantages de la présente invention est que l'on transforme la quasi-totalité de l'alcool en ester de d'acide carboxylique.

Ainsi, une quantité moindre d'alcool est présente dans la phase vapeur en tête de colonne comprenant majoritairement l'ester d'acide carboxylique, de préférence de l'ester d'acide acétique *(F₂).* En conséquence, on consomme moins d'énergie pour la séparation de l'alcool et de l'ester d'acide carboxylique.

Les exemples qui suivent, illustrent l'invention sans toutefois la limiter.

Dans les exemples, on définit le taux de transformation (TT) qui correspond au rapport entre le nombre de substrat transformées et le nombre de moles de substrat engagées.

### Exemples

Pour une meilleure compréhension, les exemples ci-après sont décrits en référence à la figure 1. Les exemples suivants sont donnés pour une production finale de 100 kg/h d'acétate d'éthyle.

### Exemple 1 (Ex 1) :

On alimente un réacteur d'estérification 1 par introduction simultanée d'un flux liquide d'éthanol 6 avec un débit de 56 kg/h et d'un flux liquide d'acide acétique 7 avec un débit de 69 kg/h. Le débit total du flux liquide d'alimentation d'alimentation est appelé débit *(F₀)*.

On introduit également dans le réacteur d'estérification 1 2,3 kg d'acide méthanesulfonique comme catalyseur. Le réacteur d'estérification 1 a une capacité de 230 kg.

De l'acide méthanesulfonique est régulièrement injecté et purgé de manière à maintenir l'activité catalytique.

La température d'entrée du réacteur est de 117°C et la pression dans le réacteur est régulée de telle sorte que le mélange réactionnel dans le réacteur est liquide, soit une pression en tête de réacteur de 2 bars absolus. Le réacteur est adiabatique.

Le flux liquide de sortie du réacteur d'estérification *(F₁)* alimente ensuite une colonne d'estérification 2. Cette colonne comprend 16 plateaux théoriques. Elle fonctionne à une pression de 1,5 bars absolus et à une température en pied de colonne de 118°C.

Le flux provenant du pied de la colonne d'estérification *(F₅)* et contenant majoritairement de l'acide acétique est recyclé vers l'entrée du réacteur d'estérification 1.

Le recyclage dudit flux (*F₅*) est effectué de telle manière que le ratio molaire acide acétique sur éthanol soit de 16. Dans ces conditions, le ratio massique entre le débit de recyclage *(F₅)* et le débit d'alimentation *(F₀)* est de 12.

Le flux en tête de colonne *(F₂)* contenant majoritairement l'acétate d'éthyle, l'eau de réaction et 2,0 % en poids d'éthanol non converti est condensé dans un échangeur 4 puis envoyé dans un décanteur 5 dans lequel deux phases sont séparées.

La phase aqueuse *(F₈)* contient majoritairement de l'eau et minoritairement de l'éthanol et de l'acétate d'éthyle. Cette phase aqueuse *(F₈)* est envoyée vers une colonne de distillation en vue de séparer l'eau et un flux comprenant majoritairement l'éthanol non converti et l'acétate d'éthyle qui est recyclé au réacteur d'estérification.

La phase organique *(F₆+F₉)* en sortie de décanteur 5 contient majoritairement de l'acétate d'éthyle et, à saturation, de l'eau et de l'éthanol. Une partie *(F₆)* de cette phase organique est renvoyée vers la tête de colonne pour assurer le reflux avec un taux de reflux *(F₆)*/*(F₉)* de 3,6.

L'autre partie *(F₉)* de la phase organique est ensuite distillée de manière à éliminer l'eau et l'éthanol qu'il contient pour obtenir la qualité désirée du produit final. La teneur en éthanol dans l'acétate d'éthyle après cette distillation finale est inférieure à 0,03 % en poids.

### Exemple comparatif 2 (Ex comp 2) :

On reproduit le procédé ci-dessus décrit dans l'exemple 1 pour un ratio molaire acide acétique/alcool de 7.

### Exemple 3 (Ex 3) :

On reproduit le procédé ci-dessus décrit dans l'exemple 1 pour un ratio molaire acide acétique/alcool de 25.

### Exemple comparatif 4 (Ex comp 4) :

On reproduit le procédé ci-dessus décrit dans l'exemple 1 pour un ratio molaire acide acétique/alcool de 10.

### Exemple comparatif 5 (Ex comp 5) :

On reproduit le procédé ci-dessus décrit dans l'exemple 1 pour un ratio molaire acide acétique/alcool de 12.

### Exemple comparatif (Ex comp) :

On reproduit le procédé ci-dessus décrit dans l'exemple 1 pour un ratio molaire acide acétique/alcool de 5.

### Résultats :

Les résultats sont présentés dans le tableau ci-dessous dans lequel la comparaison entre les exemples s'entend tous les autres paramètres étant égaux par ailleurs. On a mesuré la consommation de vapeur globale de l'ensemble de l'installation pour chacun des exemples.

| Paramètres | Unité | Ex comp | Ex comp2 | Ex comp4 | Ex comp5 | Ex 1 | Ex 3 |
|---|---|---|---|---|---|---|---|
| Ratio acide acétique / éthanol | mol/mol | 5 | 7 | 10 | 12 | 16 | 25 |
| *(F₅)*/*(F₀)* | kg/kg | 2,8 | 4,4 | 6,7 | 8,2 | 12 | 20 |
| TT éthanol | % | 87,5 | 86,9 | 86,5 | 85,8 | 83,7 | 76,1 |
| Ethanol en tête colonne (F2) | % poids | 3,4 | 2,9 | 2,4 | 2,2 | 2,0 | 1,8 |
| Ethanol après distillation finale | % poids | 0,6 | 0,03 | 0,03 | 0,03 | 0,03 | 0,03 |
| Consommation de vapeur globale | MWh/t | 2,5 | 2,3 | 1,6 | 1,6 | 1,5 | 1,5 |

L'augmentation du ratio molaire acide acétique / éthanol à l'entrée du réacteur d'estérification permet de diminuer la consommation énergétique de la colonne de distillation finale de l'acétate d'éthyle et donc de la consommation globale de l'unité. En outre, on constate que la quantité d'éthanol dans l'acétate d'éthyle après la distillation finale est nettement supérieur pour l'exemple comparatif (vingt fois supérieur). L'augmentation du ratio molaire acide acétique / éthanol à l'entrée du réacteur d'estérification permet donc également d'améliorer la qualité finale de l'acétate d'éthyle.

## Revendications

1. Procédé de fabrication d'un ester d'acide carboxylique par réaction entre un acide carboxylique et un alcool **caractérisé en ce que**
• la réaction est conduite de telle sorte que le ratio molaire acide carboxylique/alcool est au moins égal à 16
• on met en oeuvre les étapes suivantes :
a. on fait réagir l'acide carboxylique et l'alcool, en présence d'un catalyseur acide, à une température au moins égale à 50°C, et à une pression telle que le mélange réactionnel est à l'état liquide,
b. le mélange réactionnel liquide précédemment obtenu est ensuite soumis à une opération de distillation afin d'obtenir :
o un flux vapeur comprenant majoritairement l'ester de l'acide carboxylique, en tête de distillation et,
o un milieu comprenant majoritairement de l'acide carboxylique en pied de distillation,
c. on soutire le milieu en pied de distillation et on le réintroduit en début de réaction a ;
et **en ce que** l'acide carboxylique est l'acide acétique et l'alcool est choisi parmi l'éthanol, le butanol, le n-propanol et le cyclohexanol.

2. Procédé selon la revendication 1, **caractérisé en ce que** l'alcool est l'éthanol.

3. Procédé selon la revendication 1, **caractérisé en ce que** le ratio molaire acide carboxylique/alcool est compris entre 16 et 25.

4. Procédé selon la revendication 1, **caractérisé en ce que** le procédé est un procédé en continu.

5. Procédé selon la revendication 4, **caractérisé en ce que** le milieu en pied de distillation est réintroduit en début de réaction de telle sorte que le ratio entre le débit du flux de recyclage et le débit d'alimentation du mélange réactionnel (acide carboxylique + alcool) est compris entre 4 et 20.

6. Procédé selon la revendication 1, **caractérisé en ce que** la température de la réaction est comprise entre 50 et 150°C.

7. Procédé selon la revendication 6, **caractérisé en ce que** la réaction est conduite à une pression absolue comprise entre 0,5 et 5 bars absolus.

8. Procédé selon la revendication 1, **caractérisé en ce que** la température en pied de distillation est comprise entre 50 et 150°C.

9. Procédé selon la revendication 8, **caractérisé en ce que** la pression définie en tête de distillation est comprise entre 0,5 et 5 bars absolus.

10. Procédé selon la revendication 1, **caractérisé en ce que** le catalyseur est un acide protonique fort.

11. Procédé selon la revendication 10, **caractérisé en ce que** le catalyseur est un catalyseur homogène choisi parmi l'acide sulfurique, l'acide sulfonique et leurs mélanges.

12. Procédé selon la revendication 10, **caractérisé en ce que** le catalyseur est un catalyseur hétérogène choisi parmi les résines sulfoniques.

## Patentansprüche

1. Verfahren zur Herstellung eines Carboxylsäureesters durch Umsetzung einer Carboxylsäure mit einem Alkohol, **dadurch gekennzeichnet, dass**
- die Reaktion derart durchgeführt wird, dass das Molverhältnis Carboxylsäure/Alkohol mindestens gleich 16 ist,
- die folgenden Schritte durchgeführt werden:
a. Umsetzen der Carboxylsäure mit dem Alkohol in Gegenwart eines Säurekatalysators bei einer Temperatur von mindestens gleich 50 °C und bei einem derartigen Druck, dass sich das Reaktionsgemisch in flüssigem Zustand befindet,
b. anschließend Destillieren des vorher erhaltenen flüssigen Reaktionsgemischs, um Folgendes zu erhalten:
∘ einen Dampfstrom, umfassend hauptsächlich Carboxylsäureester, im Destillationskopf und
∘ ein Medium, umfassend hauptsächlich Carboxylsäure, im Destillationssumpf,
c. Abziehen des Mediums aus dem Destillationssumpf und Wiedereinführen desselben am Beginn der Reaktion a;
und dadurch, dass die Carboxylsäure Essigsäure ist, und der Alkohol ausgewählt ist aus Ethanol, Butanol, n-Propanol und Cyclohexanol.

2. Verfahren nach Anspruch 1, **dadurch gekennzeichnet, dass** der Alkohol Ethanol ist.

3. Verfahren nach Anspruch 1, **dadurch gekennzeichnet, dass** das Molverhältnis Carboxylsäure/Alkohol im Bereich zwischen 16 und 25 liegt.

4. Verfahren nach Anspruch 1, **dadurch gekennzeichnet, dass** das Verfahren ein kontinuierliches Verfahren ist.

5. Verfahren nach Anspruch 4, **dadurch gekennzeichnet, dass** das Medium im Destillationssumpf derart am Beginn der Reaktion wiedereingeführt wird, dass das Verhältnis zwischen der Recyclingstrommenge und der Einspeisemenge des Reaktionsgemischs (Carboxylsäure + Alkohol) im Bereich zwischen 4 und 20 liegt.

6. Verfahren nach Anspruch 1, **dadurch gekennzeichnet, dass** die Reaktionstemperatur im Bereich zwischen 50 und 150 °C liegt.

7. Verfahren nach Anspruch 6, **dadurch gekennzeichnet, dass** die Reaktion bei einem absoluten Druck im Bereich zwischen 0,5 und 5 bar absolut durchgeführt wird.

8. Verfahren nach Anspruch 1, **dadurch gekennzeichnet, dass** die Temperatur im Destillationssumpf im Bereich zwischen 50 und 150 °C liegt.

9. Verfahren nach Anspruch 8, **dadurch gekennzeichnet, dass** der im Destillationskopf definierte Druck im Bereich zwischen 0,5 und 5 bar absolut liegt.

10. Verfahren nach Anspruch 1, **dadurch gekennzeichnet, dass** der Katalysator eine starke protonische Säure ist.

11. Verfahren nach Anspruch 10, **dadurch gekennzeichnet, dass** der Katalysator ein homogener Katalysator ist, ausgewählt aus Schwefelsäure, Sulfonsäure und deren Gemischen.

12. Verfahren nach Anspruch 10, **dadurch gekennzeichnet, dass** der Katalysator ein heterogener Katalysator ist, ausgewählt aus Sulfonharzen.

## Claims

1. Process for manufacturing a carboxylic acid ester by reaction between a carboxylic acid and an alcohol, **characterized in that**
• the reaction is carried out so that the carboxylic acid/alcohol molar ratio is at least equal to 16,
• the following steps are used:
a. the carboxylic acid and the alcohol are reacted, in the presence of an acid catalyst, at a temperature at least equal to 50°C, and at a pressure such that the reaction mixture is in the liquid state;
b. the liquid reaction mixture obtained previously is then subjected to a distillation operation in order to obtain:
∘ a vapor stream comprising predominantly the carboxylic acid ester, at the distillation top; and
∘ a medium comprising predominantly the carboxylic acid, at the distillation bottom; and
c. the medium at the distillation bottom is withdrawn and reintroduced at the start of reaction a;
and **in that** the carboxylic acid is acetic acid and the alcohol is chosen from ethanol, butanol, n-propanol and cyclohexanol.

2. Process according to Claim 1, **characterized in that** the alcohol is ethanol.

3. Process according to Claim 1, **characterized in that** the carboxylic acid/alcohol molar ratio is between 16 and 25.

4. Process according to Claim 1, **characterized in that** the process is a continuous process.

5. Process according to Claim 4, **characterized in that** the medium at the distillation bottom is reintroduced at the start of the reaction so that the ratio between the flow rate of the recycle stream and the feed flow rate of the reaction mixture (carboxylic acid + alcohol) is between 4 and 20.

6. Process according to Claim 1, **characterized in that** the reaction temperature is between 50 and 150°C.

7. Process according to Claim 6, **characterized in that** the reaction is carried out at an absolute pressure between 0.5 and 5 bar absolute.

8. Process according to Claim 1, **characterized in that** the temperature at the distillation bottom is between 50 and 150°C.

9. Process according to Claim 8, **characterized in that** the defined pressure at the distillation top is between 0.5 and 5 bar absolute.

10. Process according to Claim 1, **characterized in that** the catalyst is a strong protonic acid.

11. Process according to Claim 10, **characterized in that** the catalyst is a homogeneous catalyst chosen from sulfuric acid, sulfonic acid and mixtures thereof.

12. Process according to Claim 10, **characterized in that** the catalyst is a heterogeneous catalyst chosen from sulfonic resins.
